# EUROPEAN PATENT SPECIFICATION

(11) **EP 1 228 768 B1**
(45) Date of publication and mention of the grant of the patent: **03.06.2009**
(21) Application number: 01130953.1
(22) Date of filing: 28.12.2001
(51) Int. Cl.: A61K 39/395, G01N 33/53, C12N 5/20, A61P 9/10, C07K 16/28

(54) **Medicament for the protection against thrombotic diseases**
Medikament zum Schutz gegen thrombotische Krankheiten
Médicament pour la protection contre les maladies thrombotiques

(30) Priority: 23.01.2001 EP 01101406
(43) Date of publication of application: 07.08.2002
(73) Proprietor: Nieswandt, Bernhard, Dr., 58285 Gevelsberg (DE)
(72) Inventor: Nieswandt, Bernhard, Dr., 58285 Gevelsberg (DE)
(74) Representative: Binsack, Beate

(56) References cited:
- WO-A-00/68377
- WO-A-01/00810
- NIESWANDT BERNHARD ET AL: "Expression and function of the mouse collagen receptor glycoprotein VI is strictly dependent on its association with the FcRgamma chain." JOURNAL OF BIOLOGICAL CHEMISTRY, vol. 275, no. 31, 4 August 2000 (2000-08-04), pages 23998-24002, XP002174650 ISSN: 0021-9258
- SCHULTE VALERIE ET AL: "Evidence for two distinct epitopes within collagen for activation of murine platelets." JOURNAL OF BIOLOGICAL CHEMISTRY, vol. 276, no. 1, 5 January 2001 (2001-01-05), pages 364-368, XP002174651 ISSN: 0021-9258
- GIBBINS JONATHAN M ET AL: "Glycoprotein VI is the collagen receptor in platelets which underlies tyrosine phosphorylation of the Fc receptor gamma-chain" FEBS LETTERS, ELSEVIER SCIENCE PUBLISHERS, AMSTERDAM, NL, vol. 413, no. 2, 1997, pages 255-259, XP002143941 ISSN: 0014-5793
- CLEMETSON JEANNINE M ET AL: "The platelet collagen receptor glycoprotein VI is a member of the immunoglobulin superfamily closely related to FcalphaR and the natural killer receptors." JOURNAL OF BIOLOGICAL CHEMISTRY, vol. 274, no. 41, 8 October 1999 (1999-10-08), pages 29019-29024, XP002143944 ISSN: 0021-9258
- NIESWANDT BERNHARD ET AL: "Long-term antithrombotic protection by in vivo depletion of platelet glycoprotein VI in mice." JOURNAL OF EXPERIMENTAL MEDICINE, vol. 193, no. 4, 19 February 2001 (2001-02-19), pages 459-469, XP002174652 ISSN: 0022-1007

## Description

Subject of the invention is a medicament for the protection against thrombotic diseases which comprises an antibody directed against the platelet collagen receptor glycoprotein (GP)VI; which binds to the same epitop of the collagen receptor on thrombocytes as the monoclonal antibody JAQ1, obtainable from the hybridoma cell line with the deposition number DSM ACC 2487.

Platelet aggregation is a key mechanism for normal hemostasis limiting blood loss after tissue trauma (1;2), but may lead to arterial occlusion in the setting of atherosclerosis and precipitate diseases such as myocardial infarction (3;4). Arterial thrombosis is often initiated by abrupt disruption of the atherosclerotic plaque and deposition and activation of platelets on the subendothelial layers (4;5). Although several of the macromolecular components of the subendothelial layer such as laminin, fibronectin, and von Willebrand factor (vWf), all provide a suitable substrate for platelet adhesion, fibrillar collagen is considered the most thrombogenic constituent of the vascular subendothelium since it not only supports platelet adhesion but is also a strong activator of platelets (6;7). The interaction between platelets and collagen involves firstly adhesion and, subsequently, activation leading to second phase adhesion, secretion, and ultimately aggregation (8;9). Besides GPIb-IX-V, which indirectly interacts with collagen via von Willebrand factor (10), several collagen receptors have been identified on platelets, including integrin α₂β₁ (11), and the nonintegrin GPVI (12). It is presently accepted that integrin α₂β₁ is the major receptor supporting platelet adhesion to collagen, whilst GPVI mediates activation (13-15). The very recent cloning of human and mouse GPVI showed that this receptor is a 60-65 kDa type I transmembrane glycoprotein belonging to the immunoglobulin superfamily (16;17) that forms a complex with the FcR γ-chain at the cell surface in human and mouse platelets (14;15;18). Signaling through GPVI occurs via a similar pathway to that used by immunoreceptors (19) as revealed by the tyrosine phosphorylation of the FcR γ-chain immunoreceptor tyrosine-based activation motif (ITAM) by a src-like kinase (20;21). GPVI-deficient patients suffer from a mild bleeding diathesis and their platelets show severely impaired responses to collagen (12;22). Furthermore, platelets from FcR γ-chain deficient mice, which lack GPVI (15), also fail to aggregate in response to collagen (14;19) but major bleeding has not been reported to occur in these mice .

It has, therefore, been proposed that GPVI may have a central function as collagen receptor for activation of human platelets. In mice, similar mechanisms seem to exist as platelets from FcRγ chain-deficient mice do not aggregate in response to collagen. In the International patent application WO 01/00810 antibodies against GPVI are already described which, however, are not known to irreversibly eliminate GPVI.

The function of GPVI has now been further investigated in control and FcRγ chain-deficient mice with an unique monoclonal antibody (mAb) against GPVI (JAQ1).

The results of these investigations can be summarized as follows:

On wild type platelets, JAQ1 inhibited platelet aggregation induced by collagen, but not platelet aggregation induced by PMA (phorbol-12-myristate-13-acetate)or thrombin. Crosslinking of bound JAQ1, on the other hand, induced aggregation of wild type, but not FcRγ-chain-deficient platelets. JAQ1 stained platelets and megakaryocytes from wild-type but not FcRγ-chain-deficient mice. Furthermore, JAQ1 recognized GPVI (approximately 60 kD) in immunoprecipitation and Western blot experiments with wild-type, but not FcRγ-chain-deficient platelets. These results strongly suggest that GPVI is the collagen receptor responsible for platelet activation in mice and demonstrate that the association with the FcRγ-chain is critical for its expression and function.

Further studies clearly showed that JAQ1 cross-reacts with human GPVI (Nieswandt B, unpublished results).

Based on these results, it has now been found that a medicament is effective against thrombotic diseases if it comprises an active principle that induces an irreversible inactivation or degradation of a collagen receptor on thrombocytes. This active principle may be a chemical compound or a monoclonal or polyclonal antibody. A preferred monoclonal antibody is JAQ1 and the preferred collagen receptor is platelet GPVI. If the monoclonal antibody JAQ1 is used it should be a humanized monoclonal antibody JAQ1. The hybridoma cell line secreting JAQ1 has been deposited under DSM ACC 2487 at the Deutsche Sammlung von Mikroorganismen und Zellkulturen GmbH in Braunschweig in accordance with the Budapest Treaty.

A further object of the invention is a diagnostic agent containing the labelled monoclonal or polyclonal antibody directed against the GPVI epitope, as defined by JAQ1, for the determination of the expression rate of the collagen receptor GPVI. Patients with higher than normal levels will thus be recognized as being threatened by thrombotic complications, whereas patients with lower than normal GPVI levels are jeopardized by bleeding events.

The antibody JAQ1 may be labelled by any conventional method all of which are available to the expert in the field of diagnostics. It may be radio-labelled, fluorescence-labelled, enzyme-labelled or may contain any other marker which allows the detection of the antibody on cells or in tissue. The diagnostic procedure may be performed as follows:
a) a sample of diluted blood of the patient is incubated with fluorescence-labelled JAQ1 for 15 minutes at room temperature and the platelets are directly analysed by flow cytometry.
b) a sample of blood of the patient is fixed on a solid carrier and subsequently treated with the labelled antibody JAQ1 alone or in mixture with unlabeled antibody JAQ1 followed by the detection of the labelled antibody by conventional methods.

These and other known alternative diagnostic procedures may be performed. Most suitable is the use of fluorescence-labelled monoclonal antibody JAQ1 in flow cytometry.

The following experiments have been performed:

### Materials and Methods

*Animals.* Specific-pathogen-free mice (NMRI) 6 to 10 weeks of age were obtained from Charles River (Sulzfeld, Germany) and kept in our animal facilities.

*Chemicals.* Anesthetic drugs xylazine (Rompun®) and ketamine (Imalgene 1000®) were delivered from Bayer (Leverkusen, Germany) and Mérial (Lyon, France), respectively. Immobilized papain (Pierce, Rockford, IL, USA), high molecular weight heparin, ADP, phorbol-12-myristate-13-acetate (PMA), (all from Sigma, Deisenhofen, Germany), FITC-labeled Annexin V (Boehringer Mannheim, Germany), and collagen (Kollagenreagent Horm, Nycomed, Munich, Germany) were purchased. CRP (GKO-(GPO)₁₀-GKOG) (single letter amino acid code where O=hydroxyproline) and convulxin were kindly provided by S.P. Watson (Oxford, U.K). FITC-labeled convulxin was a generous gift from M. Jandrot-Perrus (Paris, France).

*Antibodies.* The rat anti-mouse P-selectin mAb RB40.34 was kindly provided by D. Vestweber (Münster, Germany). Polyclonal rabbit antibodies to human fibrinogen and vWF were purchased from DAKO (Glostrup, Denmark) and were modified in our laboratories. Rabbit-anti fluorescein isothiocyanate (FITC)-HRP was from DAKO. Monoclonal antibodies against the integrin α2 and β1 subunits were from Pharmingen. All other antibodies were generated, produced, and modified in our laboratories and have been described (23;24). Modification of antibodies: Fab fragments from JAQ1 were generated by 12-hour incubation of 10 mg/ml mAb with immobilized papain (Pierce), and the preparations were then applied to an immobilized protein A column followed by an immobilized protein G column (Pharmacia) to remove Fc fragments and any undigested IgG. The purity of the Fab fragments was checked by sodium dodecyl sulphate-polyacrylamide gel electrophoresis (SDS-PAGE) and silver staining of the gel. F(ab)₂ fragments from JON/A (anti-mouse GPIIb/IIIa) were generated as described (24).

*Platelet preparation and counting.* Mice were bled under ether anesthesia from the retroorbital plexus. Blood was collected in a tube containing 10% (v/v) 0.1 M sodium citrate or 7.5 U/ml heparin and platelet rich plasma was obtained by centrifugation at 300g for 10 minutes at room temperature (RT). For determination of platelet counts, blood (20 µl) was obtained from the retroorbital plexus of anesthetized mice using siliconized microcapillaries and immediately diluted 1:100 in Unopette kits (Becton Dickinson, Heidelberg, Germany). The diluted blood sample was allowed to settle for 20 minutes in an Improved Neubauer haemocytometer (Superior, Bad Mergentheim, Germany), and platelets were counted under a phase contrast microscope at x 400 magnification.

*Immunoblotting.* Platelets (3 x 10⁸) were washed three times with PBS and subsequently solubilized in 0.3 ml lysis buffer (Tris-buffered saline containing 20 mM Tris/HCl, pH 8, 150 mM NaCI, 1 mM EDTA, 1 mM phenylmethylsulfonyl fluoride, 2 µg/ml aprotinin, 0.5 µg/ml leupeptin, and 0.5% Nonidet P-40, all from Boehringer Mannheim) for 30 min at 4°C. Cell debris was removed by centrifugation (15,000g, 10 minutes) and the whole-cell extract was run on a SDS-PAGE gel under non-reducing conditions and transferred onto a PVDF membrane. The membrane was first incubated with 5 µg/ml FITC-labeled primary antibody followed by rabbit anti-FITC-horseradish peroxidase (1 µg/ml). Proteins were visualized by ECL.

*2D-electrophoresis.* Washed platelets were peletted and resuspended in Tris 20 mM, pH 7.5, EDTA 2mM, sucrose 0.25 M. Platelets were solubilized by addition of 4 vol of Urea 8.75 M, Thiourea 2.5 M, DTT 25 mM, Triton X100 1.25 % and ampholytes 3-10, 0.75%. Two-dimensional gel electrophoresis (2D-E) was carried out as described (25). Briefly, IEF was carried out with commercially available immobilized pH gradient (linear pH gradient 3-10, 7 cm length), using the Protean IEF Cell apparatus (Biorad, Marnes-la-Coquette, France). The gels were rehydrated in the presence of the samples (platelet lysates corresponding to 5x10⁷ platelets) for 16 h and focused for 20.000 Vh. After IEF, the gel strips were incubated at room temperature in solutions containing DTT and then iodoacetamide as described (26). The gels were then subjected to the second-dimensional run and silver stained.

*Aggregometry.* To determine platelet aggregation, light transmission was measured using prp (200 µl with 0.5 x 10⁶ platelets/µl). Transmission was recorded on a Fibrintimer 4 channel aggregometer (APACT Laborgeräte und Analysensysteme, Hamburg, Germany) over ten minutes and was expressed as arbitrary units with 100% transmission adjusted with plasma. Platelet aggregation was induced by addition of collagen (5 - 50 µg/ml), PMA (50 ng/ml), or ADP (10 µM).

*Flow cytometry.* Heparinized whole blood was diluted 1:30 with modified Tyrodes-HEPES buffer (134 mM NaCI, 3.04 mM Na₂HPO₄, 2.9 mM KCI, 12 mM NaHCO₃, 20 mM HEPES, 5 mM glucose, 1 mM MgCl₂, pH 6.6) and left for 30 min at 37°C prior to stimulation. Samples were stimulated with the indicated concentrations of ADP or CRP for 2 min at RT, stained with fluorophore-labeled mAbs for 10 min at RT, and directly analyzed on a FACScan (Beckton Dickinson, Heidelberg). Flow cytometric analysis of Annexin V-FITC binding to resting and activated (combination of 50 µg/ml collagen and 0.01 U/ml thrombin) platelets was measured according to the instructions of the manufacturer.

*In vivo experiments.* Antibodies (in 200 µl PBS) were injected intraperitoneally. Thromboembolism induced by collagen and epinephrine: Mice were anesthetized by intraperitoneal injection of 150 µl of a mixture of 0.08% xylazine base (Rompun, Bayer, Germany) and 1.6% ketamine (Imalgen 1000, Mérial, France). Anesthetized mice received a mixture of collagen (0.8 mg/kg) and epinephrine (60 µg/kg) injected into the jugular vein (27). The incisions of surviving mice were stitched, and they were allowed to recover. Necroscopy and histological studies were performed on lungs fixed in 4% formaldehyde and paraffin sections were stained with hematoxylin/eosin. Bleeding time experiments: Mice were anesthetized and 3 mm of tail tip was amputated with a scalpel. The tail was then blotted with filter paper every 15s until the paper was no longer blood-stained (28). Where necessary, bleeding was manually stopped at the 10 min-time point to prevent death. Experiments were conducted in accordance to the regulations of the local authorities.

*Immunohistochemistry.* Acetone-fixed cryosections (6 µm) were blocked (5% normal goat serum, 5 mg/ml bovine serum albumin, BSA in PBS) for 30 min at RT. HRP-conjugated p0p1 (anti-mouse GPIb-IX (23)) was added at a final concentration of 2 µg/ml for 90 min and the AEC substrate was added after the three washing steps. The sections were then counterstained with hematoxilin.

*Platelet adhesion.* Collagen (2 µg) in 100 µl PBS was immobilized on F96-MaxiSorp plates (Nunc, Wiesbaden, Germany) at 4°C overnight. The plates were then saturated with 1 mg/ml BSA in PBS for 3 h at 37°C and washed with PBS. Washed platelets in Tyrode's-albumin buffer (10⁶ / well) were incubated in the wells for up to 45 min. The plates were washed three times with PBS and then incubated with HRP-labeled anti-GPIb-IX (p0p1) for 30 min at room temperature, and TMB was added to each well after 3 washing steps. The reaction was stopped by addition of 2 N H₂SO₄ after 10 min. Absorbance at 450 nm was recorded on a Multiskan MCC/340 (Labsystems, Lugano, Switzerland). The results can be summarized as follows:

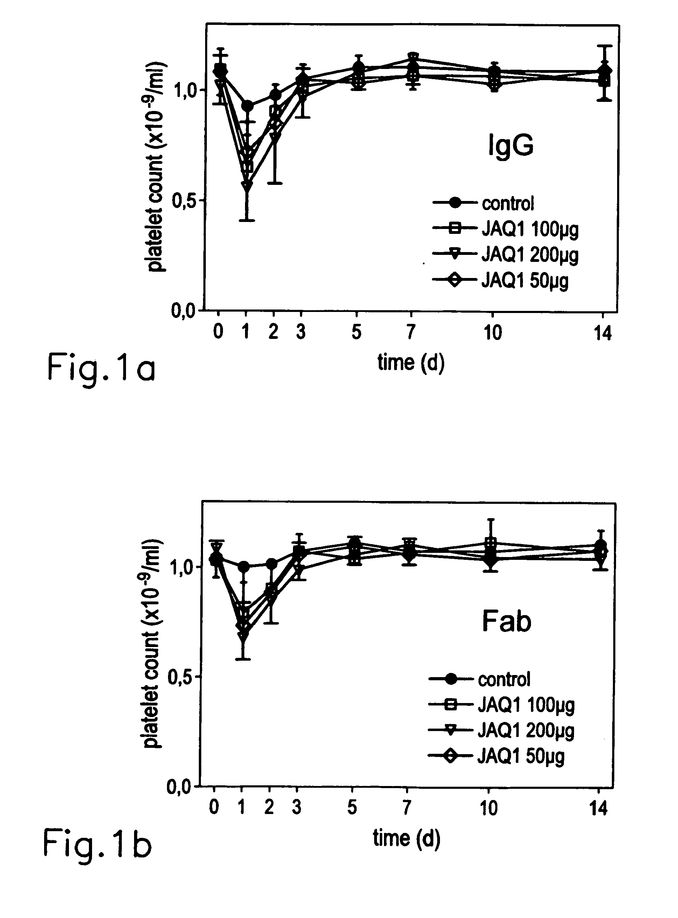

In the current study, we investigated the antithrombotic effects of JAQ1 in vivo. Injection of JAQ1 (100 µg) caused mild and transient thrombocytopenia with a maximum drop of platelet counts of approximately 34 ± 7.4 % on day 1 and a return to normal after 72h where they remained for at least 11 more days (Fig.1a). Injection of higher (200 µg) or lower (50 µg) doses had comparable effects on platelet counts (Fig.1a). The transient drop of platelet counts was not Fc-dependent as Fab fragments of JAQ1 had similar effects (Fig.1b). JAQ1-treated mice did not show any signs of anaphylactic reactions as known to be induced by anti-GPIIb/IIIa mAbs (30) and did not develop spontaneous bleeding for at least three weeks. JAQ1 was immunohistochemically detectable on splenic and bone marrow-derived megakaryocytes 3 h after antibody injection, demonstrating that the mAb reached these cells in both organs.

### JAQ1 treatment abolishes platelet responses to collagen and collagen related peptides ex vivo for at least two weeks

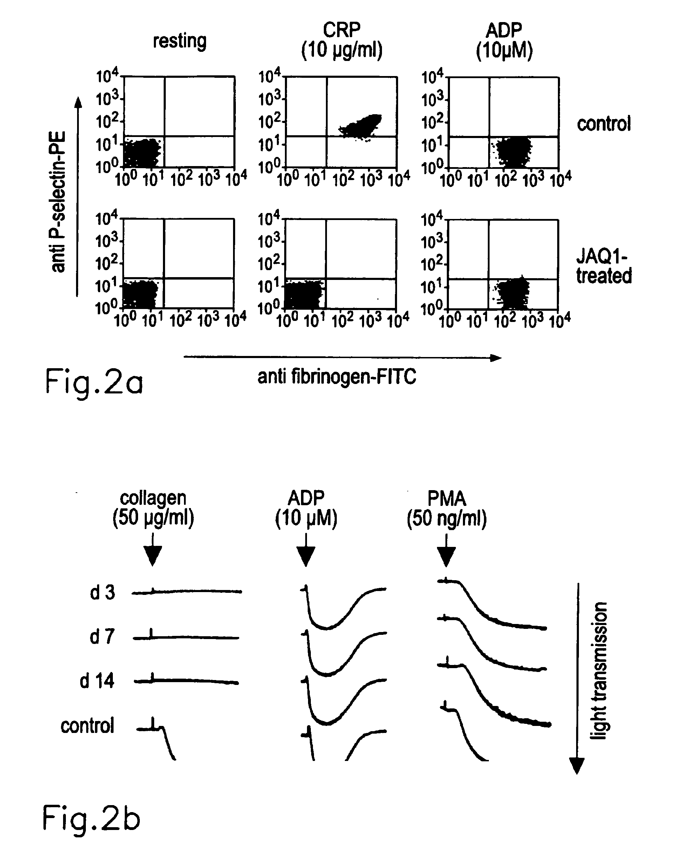

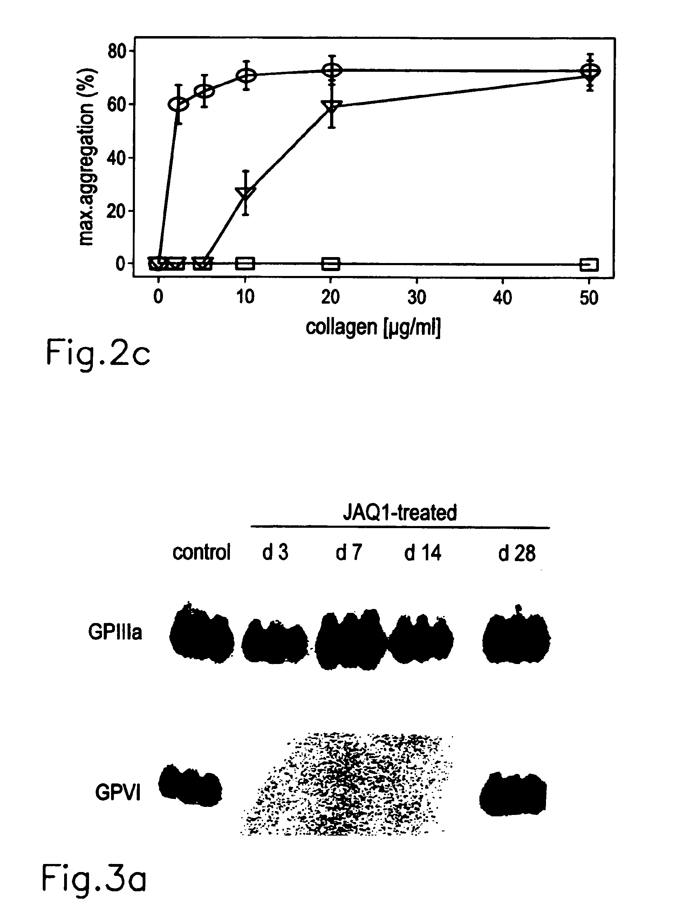

The effect of JAQ1 on circulating platelets was studied ex vivo at different time points after antibody injection. The basal surface expression of the major glycoprotein (GP) receptors GPIIb/IIIa and GPIb-IX-V, CD9, and integrin α₂β₁ was unchanged as compared to control platelets at 3, 7, and 14 days after antibody injection (Table 1). At no time after antibody injection did circulating platelets show any signs of activation, as demonstrated by the lack of surface bound fibrinogen and surface expressed P-selectin (Table 1). On days 3, 7, and 14, platelets from JAQ1-treated mice were resistant towards activation with the collagen related peptide (CRP up to 30 µg/ml), which is known to be a strong GPVI-specific platelet agonist (31) (Fig.2a). In contrast, ADP induced normal activation (fibrinogen binding) of these platelets. Furthermore, platelets from JAQ1-treated mice were completely resistant to activation with collagen at concentrations of up to 50 µg / ml ex vivo and this profound inhibitory effect also lasted for at least 14 days upon a single injection of 100 µg JAQ1 (Fig.2b). In contrast to collagen, ADP and PMA induced normal aggregation of these platelets, indicating that JAQ1 specifically blocked GPVI-dependent platelet activation pathways whereas other functions were not affected. In vitro, saturating concentrations of JAQ1 (20 µg/ml) only displayed a limited inhibitory effect on collagen-induced platelet aggregation which could be overcome when collagen concentrations higher than ~7 µg/ml were used (Fig. 2c), confirming earlier results (29).

### JAQ1 induces the loss of GPVI on circulating platelets in vivo

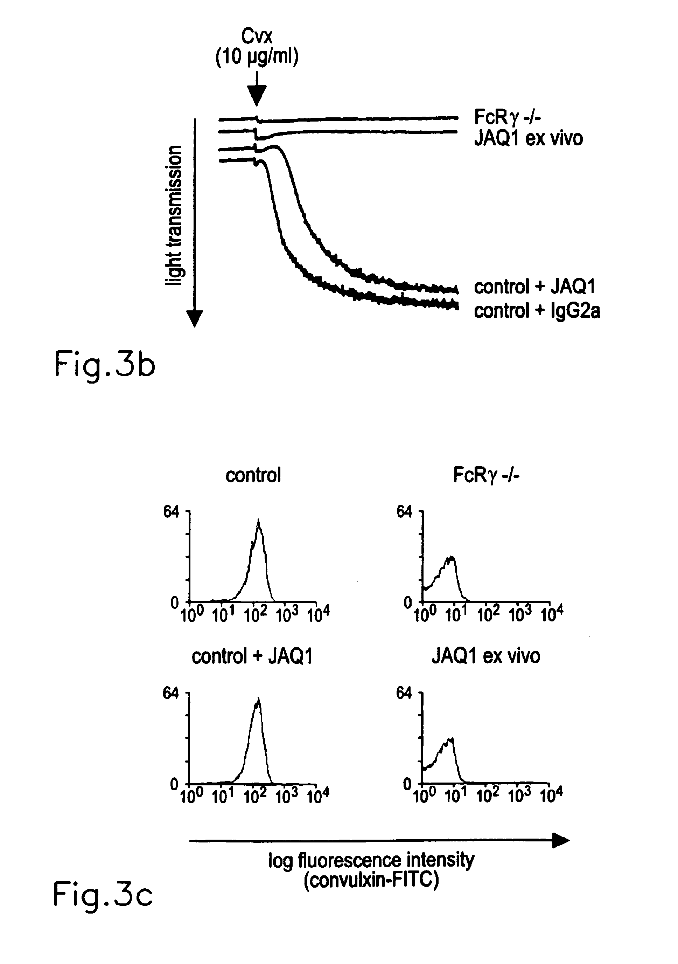

The discrepancy between the inhibitory effect of JAQ1 on collagen-induced aggregation in vitro and ex vivo was surprising and suggested that mechanisms other than pure blockage of an epitope on GPVI must be involved. Therefore, the next step was to test platelets from JAQ1-treated mice for the presence of GPVI in a Western blot analysis of whole cell lysates. As shown in Fig. 3a, GPVI was not detectable in platelets from JAQ1-treated mice for at least 14 days upon a single injection of JAQ1, whereas GPIIIa was present in normal amounts at any time point. In contrast, in all mice tested, new platelets expressing functional GPVI were detectable after 28 days. To further assess the absence of GPVI on platelets from JAQ1-treated mice, we used the GPVI-specific snake venom toxin convulxin (32). As shown in Fig. 3b, convulxin did not induce aggregation of platelets from JAQ1-treated mice on day 3, 7, and 14, whereas it induced aggregation of control platelets in the presence or absence of saturating amounts of JAQ1. Furthermore, flow cytometric analysis demonstrated that FITC-labeled convulxin did not bind to platelets from JAQ1-treated mice (Fig. 3c). Finally, the absence of a ~ 60 kD protein with an isoelectric point of 5.6 in the platelets from JAQ1-treated mice was confirmed by 2-D gel electrophoresis. Together, these results strongly suggested that GPVI had been irreversibly inactivated and removed from these platelets in vivo.

### JAQ1-induced GPVI loss occurs rapidly in vivo and is Fc-independent

To examine the mechanisms underlying the loss of GPVI, mice were injected with biotinylated JAQ1 and the amount of surface-bound mAb was determined by flow cytometry ex vivo at early time points after injection. Interestingly, as soon as 6 hours after injection only very low levels of surface-bound JAQ1 were detectable and the signals further decreased to control after 24 and 48 h while JAQ1^{FITC} and Cvx^{FITC} bound to the platelets at no time point. These data suggested that the JAQ1-GPVI complex had been cleared from the surface of those platelets within 6h. In contrast, platelets from mice injected with a biotinylated mAb against GPV (24) constantly yielded positiv staining with FITC-labeled streptavidin. In the next step, we tested whole cell lysates from platelets of JAQ1-treated mice for the presence of GPVI and the biotinylated mAb. JAQ1 was strongly detectable in platelets 6 h after injection whereas signals markedly decreased at 24 h and even more at 48 h. A similar picture was found for GPVI, strongly suggesting that the JAQ1-GPVI complex had become internalized and was degraded within two days. In contrast to its in vivo effects, JAQ1 did not induce any detectable downregulation of surface GPVI within 6 h incubation at 37°C on washed platelets or in whole blood (heparinized or citrated), indicating that a second signal may be required to induce this effect and that this signal is absent in vitro.

To determine whether the Fc part of JAQ1 or its divalent form is required for internalization/ degradation of GPVI, mice received 100 µg Fab fragments of the mAb and the platelets were tested for the presence of GPVI after 48 h. The Fab fragments, like the intact IgG, induced the complete loss of GPVI from circulating platelets and the cells were completely resistant towards activation with CRP, collagen, or convulxin.

### GPVI-depleted platelets display reduced adhesion to collagen and abolished collagen-dependent procoagulant activity

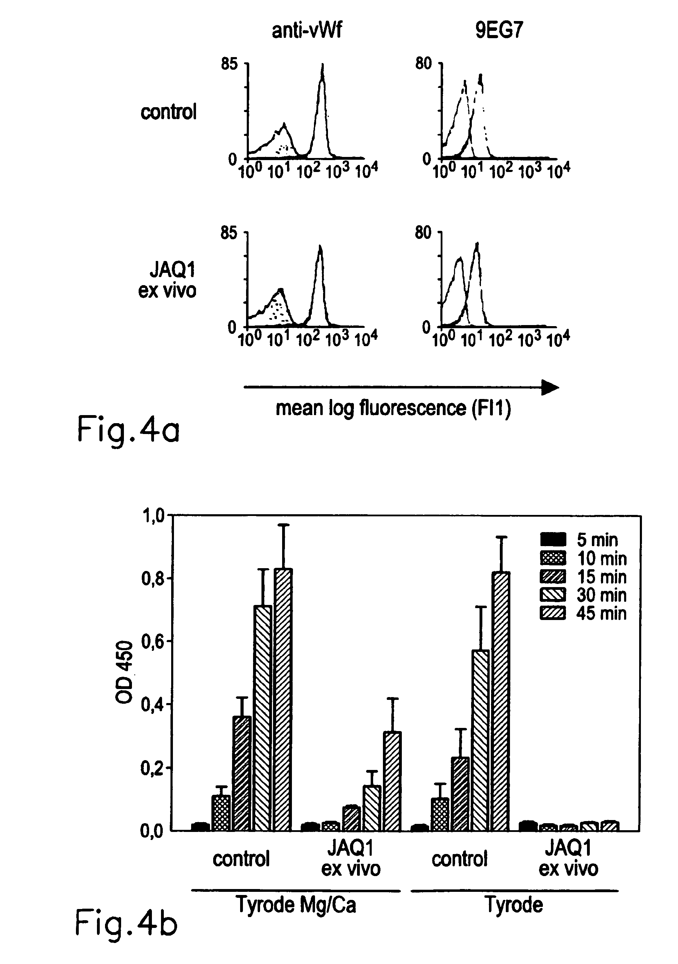

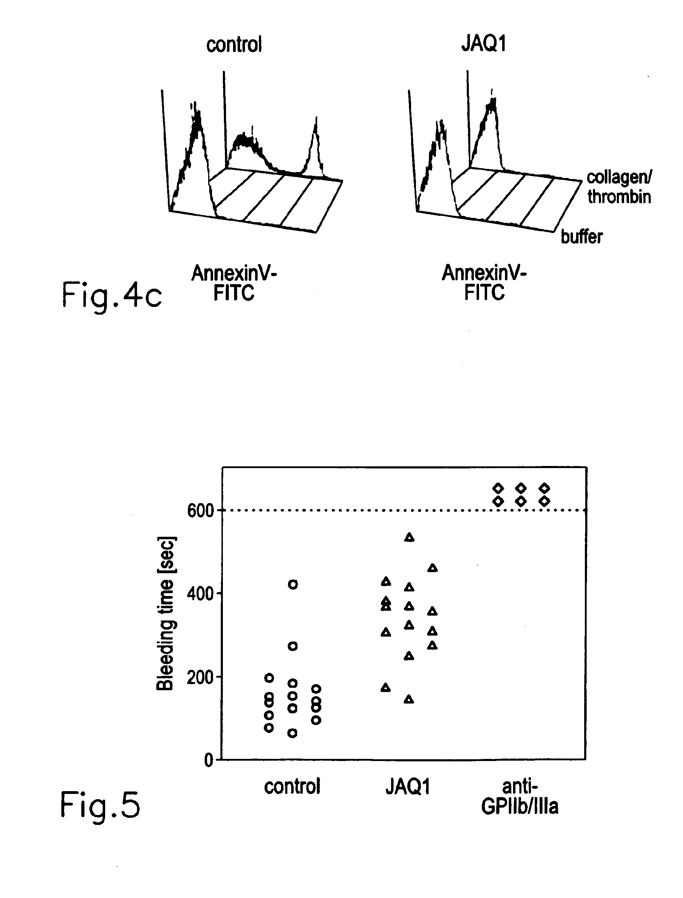

It is currently thought that GPVI is the platelet collagen receptor for activation, whilst integrin α₂β₁ and GPIb-V-IX (via vWF) mediate adhesion. As shown before (Table 1), the basal surface expression of both receptors was not influenced by the JAQ1 treatment. Further experiments demonstrated that platelets from JAQ1-treated mice bound normal levels vWF in the presence of botrocetin and thrombin induced normal activation of β₁-integrins as assessed with the mAb 9EG7, which specifically recognizes the activated form of the β1 subunit (33) (Fig. 4a). In the next step, the adhesion of platelets from JAQ1-treated mice to collagen was tested in a static assay. As shown in Fig. 4b, the adhesion of platelets from JAQ1-treated mice was strongly reduced as compared to control platelets and was abolished in the absence of extracellular free magnesium / calcium, strongly suggesting it to be mediated predominantly by integrin α₂β₁ (34). It is well known that GPVI is critically involved in the procoagulant response of platelets where stimulated platelets expose negatively charged phosphatidylserine (PS) at the plasma membrane which facilitates thrombin generation (35). Indeed, platelets from JAQ1-treated mice did not expose PS in response to a combination of collagen and thrombin on day 3, 7, and 14 after antibody injection as demonstrated by the lack of Annexin V binding (Fig. 4c).

### Anti-GPVI treatment induces long-term antithrombotic protection but only moderately increased bleeding times

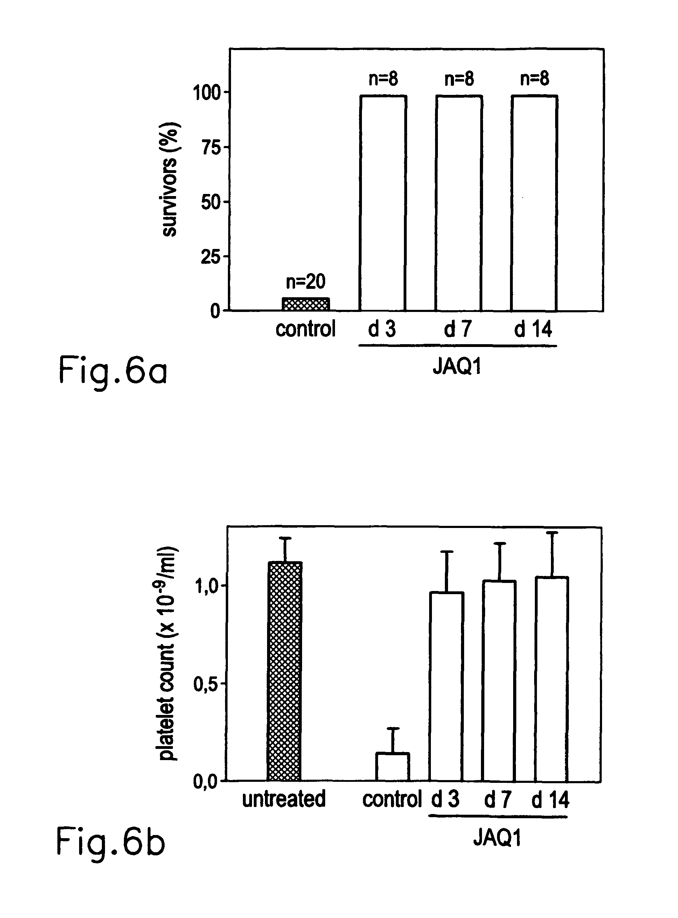

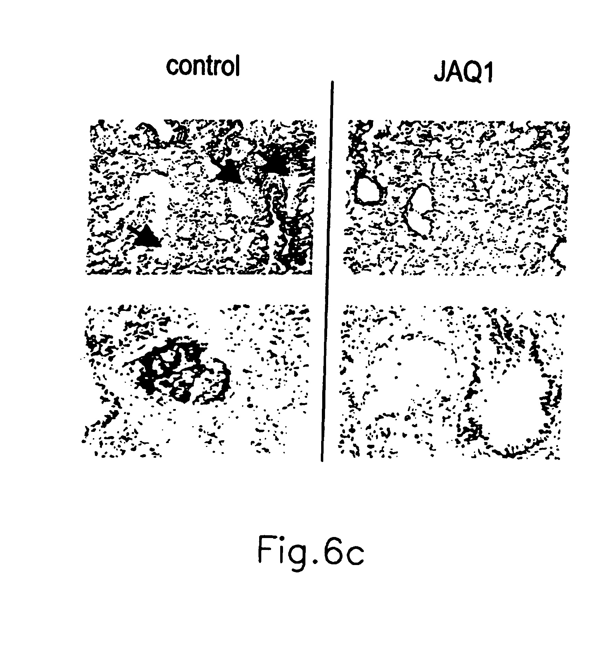

The results of the previous experiments suggested that JAQ1 specifically induced complete depletion of GPVI in platelets in vivo. To examine to which extent this specific defect influenced normal hemostasis, we determined the tail bleeding times on day 7 after a bolus injection of JAQ1 (100 µg). As shown in Fig. 5, the bleeding times were significantly increased in GPVI-depleted mice compared to control mice (330 ± 103 vs. 158 ± 89 sec., respectively), but consistently lower than in mice pre-treated with 100 µg blocking F(ab)₂ fragments against GPIIb/IIIa (24) (>600 sec.). In the next step, we examined the protective effect of JAQ1 in a model of lethal pulmonary thromboembolism induced by infusion of a mixture of collagen (0.8 mg/kg body weight) and epinephrine (60 µg/kg body weight) (27). Among control mice pre-treated with irrelevant rat IgG2a, 95% (19 of 20) died within 5 min from widespread pulmonary thrombosis and cardiac arrest. In contrast, all mice pre-treated with JAQ1 (100 µg) survived, irrespective of whether they had received the mAb 3, 7, or 14 days before challenge (n=8 per group) (Fig. 6a). While the platelet counts in JAQ1 pre-treated mice had not been influenced significantly by the infusion of collagen/epinephrine, there was a sharp decrease detectable in control mice (n=8) which was determined 3 min after induction of thromboembolism in a separate group (Fig. 6b). For histological examination, control and JAQ1 pre-treated (3, 7, and 14 days) mice received the same treatment in parallel experiments but the lungs were removed after 3 min. While the vast majority of large and small vessels were obstructed by platelet rich thrombi in the lungs of control mice, there were only very few thrombi detectable in the lungs of JAQ1 pre-treated mice (Fig. 6c).

Thus, it could be demonstrated that treatment of mice with a monoclonal antibody against GPVI results in profound long-term antithrombotic protection against collagen-dependent thromboembolism. These results confirm the proposed critical role of GPVI in collagen-induced activation of platelets in vivo and indicate that anti-GPVI agents might be effective in preventing arterial thrombosis induced by atherosclerotic plaque rupture, where platelets are thought to become activated mainly by the subendothelium under conditions of high shear stress (4;5;36). Among the matrix proteins which support platelet adhesion and subsequent activation, collagen has a critical role, at least in normal hemostasis as patients with defects in collagen receptors display mild bleeding disorders (12;37;38). Although the role of GPIb, GPIIbIIIa and their respective ligands von Willebrand factor (vWF) and fibrinogen in thrombosis are well documented (as reviewed by Z.M. Ruggeri (39)), the finding that vWF and fibrinogen double knockout mice are still able to form occlusive thrombi suggests that collagen and its platelet receptors might also have a critical role in thrombosis (40).

The profound inhibitory effect of JAQ1 in vivo was unexpected since it was based on clearing of GPVI from circulating platelets and no such specific depletion of a platelet receptor has been described to date. The complete loss of functional GPVI on circulating platelets in JAQ1-treated mice was confirmed by different approaches. Firstly, the protein was not detectable in a Western blot analysis of platelet lysates for at least two weeks (which exceeds the normal life-span of platelets (41)). Secondly, the GPVI-specific snake venom toxin convulxin, which binds to a different epitope than JAQ1 (Fig.3b, c), did not bind to platelets from JAQ1-treated mice strongly suggesting the absence of GPVI from the platelet membrane. Thirdly, a ~60 kD protein with an isoelectric point of ~5.6 (which is similar to that described for human GPVI (42)) is absent in the lysate of platelets from JAQ1-treated mice (Fig.4) and the same protein is absent in platelets from FcRγ chain-deficient mice (not shown) which are known to lack GPVI (15). Most importantly, the functional platelet responses to collagen were completely abolished by JAQ1 in vivo, whereas the mAb only has limited inhibitory effects in vitro (29), (Fig.2c). These results demonstrate that JAQ1 induced the clearing of GPVI from the surface of circulating platelets in vivo. This finding is also supported by the observation that biotinylated JAQ1 was detectable in the lysates, but not on the surface, of platelets 6 h after injection and the same was found for GPVI. Furthermore, the decreasing signals for both GPVI and JAQ1 after 24 and 48 h strongly suggest that the internalized complex was degraded in the intracellular compartments. GPVI belongs to the immunoglobulin superfamily and is closely related to immunoreceptors, some of which may become internalized when stimulated appropriately (43;44). In the case of JAQ1-GPVI it was difficult to define what the appropriate stimulus is, but it seems clear that the Fc part of the mAb is not required to induce internalization as Fab fragments produced the same effect, thereby also excluding the requirement for GPVI clustering. In vitro, JAQ1 did not induce the downregulation of GPVI from the platelet membrane (Fig.5a) suggesting that a second signal may be required for the induction of this process that is provided by other cells in vivo. This assumption may be supported by the observation that JAQ1 and Fab fragments of the mAb induced transient thrombocytopenia. The reason for this is not clear, but it might be due to weak activation of GPIIb/IIIa leading to formation of loose aggregates and their temporary sequestration to the spleen where the actual loss of GPVI may occur. Recent evidence indicates that JAQ1 recognizes an epitope identical with or in close vicinity to the CRP binding site on GPVI (29) which is regarded as the major binding site for collagen on the receptor. So far, very little is known about the cellular regulation of GPVI but in the light of the current data it seems possible that occupancy of this epitope provides a signal that finally results in downregulation of the receptor.

Irrespective of the underlying mechanism, platelets from JAQ1-treated mice were completely unresponsive towards activation with high concentrations of CRP or collagen whereas they were normally activatable with ADP or PMA. This strongly suggests that JAQ1 selectively induced a transient GPVI deficiency in mice while other membrane glycoproteins, including GPIIb/IIIa, GPIb-IX-V, CD9, and integrin α₂β₁ were not affected in expression and/or function. JAQ1-treated mice had prolonged bleeding times which confirms the important role of GPVI in normal hemostasis and correlates well with the bleeding diathesis in GPVI deficient patients (12;22). Very interestingly, one GPVI deficient patient developed highly specific antibodies against the absent receptor (45) which may be difficult to explain. Based on the results presented here, however, it is feasible to speculate that this patient may suffer from an acquired GPVI deficiency, based on autoantibody-induced clearing of GPVI from her circulating platelets.

Besides its pivotal role in collagen-induced platelet activation, GPVI is also critically involved in the procoagulant reaction of platelets (46) which was confirmed by the abolished collagen-dependent procoagulant activity of platelets from JAQ1-treated mice. This result strongly suggests that anti-GPVI treatment also modulates coagulation at sites of vascular injury. Such an anticoagulant activity has been demonstrated for GPIIb/IIIa antagonists (47), which are currently considered the most powerful inhibitors of platelet participation in thrombosis (48), as they inhibit the final common pathway of platelet aggregation, irrespective of the agonist that stimulates the cells. It has been suggested that this more or less complete inhibition of platelet function may come with a potential safety risk as platelet aggregation is also required for normal hemostasis (49). We found that JAQ1 induced significantly shorter bleeding times than blocking antibodies against GPIIb/IIIa in mice, indicating that GPVI-depleted platelets still contributed significantly to normal hemostasis in vivo. Although there is no clear correlation between the bleeding time and bleeding risk (50) it is tempting to speculate on the grounds of these results that anti-GPVI therapy might be associated with a relatively low risk of clinical hemorrhage.

The mechanisms of collagen-platelet interactions are complex and involve direct or indirect binding of collagen to several platelet receptors, including the GPIb-IX-V complex, integrin α₂β₁, GPIV, GPVI, and 65- and 85-kD proteins (51). Despite its essential role in collagen-induced activation of platelets, there has been only very limited evidence for a role of GPVI in adhesion to collagen (17) which is mainly thought to be mediated by GPIb-IX-V (via von Willebrand factor, vWf) and integrin α₂β₁. In mice, GPVI-depleted platelets expressed normal amounts of integrin α₂β₁ and β₁-integrins were normally activatable which has been reported to be a prerequisite for effective binding of collagen (Fig. 4b) (52). Indeed, GPVI-depleted platelets adhered to collagen through α₂β₁, but the extent of adhesion was strongly reduced as compared to control platelets. A similar observation has been reported with platelets from GPVI deficient patients (12;45), indicating that GPVI may be required for normal adhesion to collagen probably by supporting the activation of α₂β₁ (53). The expression of GPIb-IX-V was not affected by the JAQ1 treatment and the receptor bound normal levels of vWF in the presence of botrocetin (Fig. 4a). Together, these results suggest that platelet adhesion to collagen at sites of vascular injury may be reduced, but not blocked, by anti-GPVI treatment.

Very recent evidence suggests that GPVI is exclusively expressed in platelets and mature megakaryocytes (17;54) and this is confirmed by immunohistochemical studies with JAQ1. Therefore, the effects of anti-GPVI agents (like JAQ1) should be restricted to platelets and, very importantly, megakaryocytes. JAQ1 was detectable on megakaryocytes in spleen and bone marrow 3h after antibody injection, suggesting that the next generation of platelets was also affected by the mAb. This assumption may be confirmed by the fact that GPVI was not detectable in platelets for at least two weeks, although the normal life-span of mouse platelets is only approximately 4-5 days (41). Based on the estimated number of approximately 2 x 10⁹ platelets /mouse (10⁹ / ml blood) and a life span of the cells of 5 days, the GPVI molecules of 6 x 10⁹ platelets must be depleted to result in the absence of the receptor for 15 days. The amount of 100 µg JAQ1 (MW: 150 kd) represents ~6.7 x 10¹³ antibody molecules. Therefore, ~ 1.1 x 10⁴ antibody molecules per platelet are available to bind and deplete GPVI. Since the estimated expression rate of GPVI is only 1-2 x 10³ copies/platelet (55) 100 µg JAQ1 is sufficient to induce the observed effect.

Preliminary results show that a second injection of JAQ1 two weeks after the first injection has no influence on platelet counts, but prolongs the absence of GPVI on circulating platelets. This indicates that the second dose of the mAb affects newly differentiated megakaryocytes, but has no effect on circulating (GPVI-depleted) platelets. Thus, JAQ1 can be used to induce a GPVI knock out-like phenotype in mice for several weeks, allowing studies on platelet function in the absence of this critical activating receptor in vitro and in vivo.

### The thrombin response in JAQ1-treated mice is transiently reduced

After activation of the platelets of JAQ1-treated mice with the coagulation protease α-thrombin on day 3 after JAQ1 injection a significantly reduced thrombin response was detectable by measuring of GPIIbIIIa activation and P-selectin expression in response to increasing concentrations of α-thrombin. In contrast, this inhibition was not detectable in JAQ1-treated mice on days 7 and 14. This finding suggests that a selective inhibition of the thrombin response occurs during the early phase in platelets on anti-GPVI treatment.

To define this inhibition in more detail, the platelets from JAQ1-treated mice were analyzed on days 1, 2, 3, 4 and 5 after antibody injection. In order to minimize the transient drop of platelets counts, these mice received three injections of 33 µg JAQ1 within 2 hours. This treatment resulted in a mild decrease of platelet counts on day 1 and a return to normal on day 2 where they remained for at least 12 more days. The platelets from these mice showed a marked reduction in the thrombin response on days 1 and 2 which progressively returned to normal between days 3 and 5. In contrast, the platelets were fully activatable with ADP and PMA at any time point.

These results strongly suggested that the JAQ1-induced GPVI internalisation transiently affected the function of one ore more thrombin receptors in circulating platelets. This effect was identified to be related to a reduced activity of the PAR4 thrombin receptor as shown by flow cytometric analysis of platelets from JAQ1-treated mice stimulated with an PAR4-activating peptide.

Next, JAQ1-treated mice were subjected to a model of thrombin-dependent thromboembolism to determine the relevance of the observed effect for thrombotic processes *in vivo.* Anesthetized male NMRI mice (28-30 g body weight) received recombinant human thromboplastin (Thromborel, Dade Behring) *i.v..* This treatment is known to initiate intravascular thrombin formation which leads to platelet activation. These thrombin-activated platelets then facilitate further thrombin generation and finally intravascular thromboembolism. The i.v. injection of 150 µl/kg body weight thromboplastin resulted in 60% (12/20) mortality in control mice pre-treated with irrelevant rat IgG, whereas non (0/20) of the JAQ1-treated mice died on day 1 and 2 after antibody injection. The mortality increased to 35% (7/20) on day 3, further to 40% (8/20) on day 4, and finally reached the level of the control group on day 5 with 65% (13/20). These findings correlated well with the reduced thrombin response seen in JAQ1 treated mice *ex vivo.* The following parameters were determined two minutes after injection of 150 µl/kg body weight thromboplastin in control and JAQ1-treated mice in separate groups. Platelet consumption and plasmatic TAT concentrations were significantily reduced in JAQ1-treated mice as compared to controls. Again, this effect was strongest on days 1 and 2 after JAQ1 injection and progressively decreased between days 3 and 5.

Together, these findings demonstrate that the treatment of mice with the anti-GPVI mAb JAQ1 results in two distinct phases of platelet inhibition: During the first 3 - 4 days, the platelets show a complete inhibition of collagen responses and partial inhibition of thrombin responses and therefore a profound antithrombotic protection. After this period, the thrombin response returns to normal whereas the collagen response remains absent, resulting in a more moderate antithrombotic protection.

Taken together, these results suggest that GPVI might become an interesting target for long-term prophylaxis of ischemic cardiovascular diseases and provide the first evidence that it is possible to specifically deplete an activating glycoprotein receptor from circulating platelets in vivo. These findings may open the way for the development of a new generation of powerful, yet safe, antithrombotics.

Thus, it is an object of the present invention to provide a medicament for the protection against thrombotic diseases which comprises as active principle, an antibody, against a platelet collagen receptor that not only blocks, but irreversibly depletes the target receptor. Such a monoclonal antibody is defined by its binding to the same epitop of the collagen receptor on thrombocytes as the monoclonal antibody JAQ1. Preferably, as antibody the monoclonal antibody JAQ1 should be used. The collagen receptor is platelet GPVI. Most preferred is a medicament which contains the respective humanized monoclonal antibody for protection againts thrombotic diseases.

The monoclonal antibody JAQ1 can be humanized by standard methods which are well known to the experts in the field. Said humanized monoclonal antibody is usually administered to a patient who is jeopardized by thrombotic diseases in the form of a physiologically acceptable aqueous injection. Other forms of administration are not excluded. The monoclonal antibody will be administered in a quantity which is subject to the physical condition of the patient. The experienced medical doctor will have no difficulty to find out the optimum quantity of the monoclonal antibody for the intended purpose.

References
1. Weiss, H.J. 1975. Platelet physiology and abnormalities of platelet function (first of two parts). N EngI J Med 293:531.
2. Weiss, H.J. 1975. Platelet physiology and abnormalities of platelet function (second of two parts). N EngI J Med 293:580.
3. Fuster, V., L. Badimon, J.J. Badimon, and J.H. Chesebro. 1992. The pathogenesis of coronary artery disease and the acute coronary syndromes (1). N EngI J Med 326:242.
4. Fuster, V., L. Badimon, J.J. Badimon, and J.H. Chesebro. 1992. The pathogenesis of coronary artery disease and the acute coronary syndromes (2). N EngI J Med 326:310.
5. Conti, C.R. and J.L. Mehta. 1987. Acute myocardial ischemia: role of atherosclerosis, thrombosis, platelet activation, coronary vasospasm, and altered arachidonic acid metabolism. Circulation 75:V84.
6. Baumgartner, H.R. 1977. Platelet interaction with collagen fibrils in flowing blood. I. Reaction of human platelets with alpha chymotrypsin-digested subendothelium. Thromb Haemost 37:1.
7. Hawiger, J. 1987. Macromolecules that link platelets following vessel wall injury. Ann N Y Acad Sci 509:131.
8. Morton, L.F., A.R. Peachey, and M.J. Barnes. 1989. Platelet-reactive sites in collagens type I and type III. Evidence for separate adhesion and aggregatory sites. Biochem J 258:157.
9. Santoro, S.A., J.J. Walsh, W.D. Staatz, and K.J. Baranski. 1991. Distinct determinants on collagen support alpha 2 beta 1 integrin-mediated platelet adhesion and platelet activation. Cell Regul 2:905.
10. Savage, B., F. Almus-Jacobs, and Z.M. Ruggeri. 1998. Specific synergy of multiple substrate-receptor interactions in platelet thrombus formation under flow. Cell 94:657.
11. Santoro, S.A. and M.M. Zutter. 1995. The alpha 2 beta 1 integrin: a collagen receptor on platelets and other cells. Thromb Haemost 74:813.
12. Moroi, M., S.M. Jung, M. Okuma, and K. Shinmyozu. 1989. A patient with platelets deficient in glycoprotein VI that lack both collagen-induced aggregation and adhesion. J Clin Invest 84:1440.
13. Kehrel, B., S. Wierwille, K.J. Clemetson, O. Anders, M. Steiner, C.G. Knight, R.W. Farndale, M. Okuma, and M.J. Barnes. 1998. Glycoprotein VI is a major collagen receptor for platelet activation: it recognizes the platelet-activating quaternary structure of collagen, whereas CD36, glycoprotein IIb/IIIa, and von Willebrand factor do not. Blood 91:491.
14. Gibbins, J.M., M. Okuma, R. Farndale, M. Barnes, and S.P. Watson. 1997. Glycoprotein VI is the collagen receptor in platelets which underlies tyrosine phosphorylation of the Fc receptor gamma-chain. FEBS Lett 413:255.
15. Nieswandt, B., W. Bergmeier, V. Schulte, K. Rackebrandt, J.E. Gessner, and H. Zirngibl. 2000. Expression and function of the mouse collagen receptor glycoprotein VI is strictly dependent on its association with the FcRgamma chain. J Biol Chem 275:23998.
16. Clemetson, J.M., J. Polgar, E. Magnenat, T.N. Wells, and K.J. Clemetson. 1999. The platelet collagen receptor glycoprotein VI is a member of the immunoglobulin superfamily closely related to FcalphaR and the natural killer receptors. J Biol Chem 274:29019.
17. Jandrot-Perrus, M., S. Busfield, A.H. Lagrue, X. Xiong, N. Debili, T. Chickering, J.P. Couedic, A. Goodearl, B. Dussault, C. Fraser, W. Vainchenker, and J.L. Villeval. 2000. Cloning, characterization, and functional studies of human and mouse glycoprotein VI: a platelet-specific collagen receptor from the immunoglobulin superfamily [In Process Citation]. Blood 96:1798.
18. Tsuji, M., Y. Ezumi, M. Arai, and H. Takayama. 1997. A novel association of Fc receptor gamma-chain with glycoprotein VI and their co-expression as a collagen receptor in human platelets. J Biol Chem 272:23528.
19. Poole, A., J.M. Gibbins, M. Turner, M.J. van Vugt, J.G. van de Winkel, T. Saito, V.L. Tybulewicz, and S.P. Watson. 1997. The Fc receptor gamma-chain and the tyrosine kinase Syk are essential for activation of mouse platelets by collagen. EMBO J 16:2333.
20. Ezumi, Y., K. Shindoh, M. Tsuji, and H. Takayama. 1998. Physical and functional association of the Src family kinases Fyn and Lyn with the collagen receptor glycoprotein VI-Fc receptor gamma chain complex on human platelets. J Exp Med 188:267.
21. Briddon, S.J. and S.P. Watson. 1999. Evidence for the involvement of p59fyn and p53/56lyn in collagen receptor signalling in human platelets. Biochem J 338 ( Pt 1):203.
22. Arai, M., N. Yamamoto, M. Moroi, N. Akamatsu, K. Fukutake, and K. Tanoue. 1995. Platelets with 10% of the normal amount of glycoprotein VI have an impaired response to collagen that results in a mild bleeding tendency [published erratum appears in Br J Haematol 1995 Apr;89(4):952]. Br J Haematol 89:124.
23. Bergmeier, W., K. Rackebrandt, W. Schroder, H. Zirngibl, and B. Nieswandt. 2000. Structural and functional characterization of the mouse von Willebrand factor receptor GPIb-IX with novel monoclonal antibodies. Blood 95:886.
24. Nieswandt, B., W. Bergmeier, K. Rackebrandt, J.E. Gessner, and H. Zirngibl. 2000. Identification of critical antigen-specific mechanisms in the development of immune thrombocytopenic purpura in mice [In Process Citation]. Blood 96:2520.
25. Rabilloud, T. 2000. Detecting proteins separated by 2-D gel electrophoresis. Anal Chem 72:48A.
26. Chevallet, M., V. Santoni, A. Poinas, D. Rouquie, A. Fuchs, S. Kieffer, M. Rossignol, J. Lunardi, J. Garin, and T. Rabilloud. 1998. New zwitterionic detergents improve the analysis of membrane proteins by two-dimensional electrophoresis. Electrophoresis 19:1901.
27. DiMinno, G. and M.J. Silver. 1983. Mouse antithrombotic assay: a simple method for the evaluation of antithrombotic agents in vivo. Potentiation of antithrombotic activity by ethyl alcohol. J Pharmacol Exp Ther 225:57.
28. Carmeliet, P., J.M. Stassen, L. Schoonjans, B. Ream, J.J. van den Oord, M. De Mol, R.C. Mulligan, and D. Collen. 1993. Plasminogen activator inhibitor-1 gene-deficient mice. II. Effects on hemostasis, thrombosis, and thrombolysis. J Clin Invest 92:2756.
29. Schulte, V., D. Snell, W. Bergmeier, H. Zirngibl, S.P. Watson, and B. Nieswandt. 2000. Evidence for two distinct epitopes within collagen for activation of murine platelets. J Biol Chem 2001, 276, 364 - 368.
30. Nieswandt, B., B. Echtenacher, F.P. Wachs, J. Schroder, J.E. Gessner, R.E. Schmidt, G.E. Grau, and D.N. Mannel. 1999. Acute systemic reaction and lung alterations induced by an antiplatelet integrin gpIIb/IIIa antibody in mice. Blood 94:684.
31. Asselin, J., J.M. Gibbins, M. Achison, Y.H. Lee, L.F. Morton, R.W. Farndale, M.J. Barnes, and S.P. Watson. 1997. A collagen-like peptide stimulates tyrosine phosphorylation of syk and phospholipase C gamma2 in platelets independent of the integrin alpha2beta1. Blood 89:1235.
32. Polgar, J., J.M. Clemetson, B.E. Kehrel, M. Wiedemann, E.M. Magnenat, T.N.C. Wells, and K.J. Clemetson. 1997. Platelet activation and signal transduction by convulxin, a C-type lectin from Crotalus durissus terrificus (tropical rattlesnake) venom via the p62/GPVI collagen receptor. J Biol Chem 272:13576.
33. Lenter, M., H. Uhlig, A. Hamann, P. Jeno, B. Imhof, and D. Vestweber. 1993. A monoclonal antibody against an activation epitope on mouse integrin chain beta 1 blocks adhesion of lymphocytes to the endothelial integrin alpha 6 beta 1. Proc Natl Acad Sci U S A 90:9051.
34. Onley, D.J., C.G. Knight, D.S. Tuckwell, M.J. Barnes, and R.W. Farndale. 2000. Micromolar Ca2+ concentrations are essential for Mg2+-dependent binding of collagen by the integrin alpha 2beta 1 in human platelets. J Biol Chem 275:24560.
35. Bevers, E.M., P. Comfurius, J.L. van Rijn, H.C. Hemker, and R.F. Zwaal. 1982. Generation of prothrombin-converting activity and the exposure of phosphatidylserine at the outer surface of platelets. Eur J Biochem 122:429.
36. Lusis, A.J. 2000. Atherosclerosis. Nature 407:233.
37. Nieuwenhuis, H.K., J.W. Akkerman, W.P. Houdijk, and J.J. Sixma. 1985. Human blood platelets showing no response to collagen fail to express surface glycoprotein la. Nature 318:470.
38. Ryo, R., A. Yoshida, W. Sugano, M. Yasunaga, K. Nakayama, K. Saigo, M. Adachi, N. Yamaguchi, and M. Okuma. 1992. Deficiency of P62, a putative collagen receptor, in platelets from a patient with defective collagen-induced platelet aggregation. Am J Hematol 39:25.
39. Ruggeri, Z.M. 1997. Mechanisms initiating platelet thrombus formation [published erratum appears in Thromb Haemost 1997 Oct;78(4):1304] [see comments]. Thromb Haemost 78:611.
40. Ni, H., C.V. Denis, S. Subbarao, J.L. Degen, T.N. Sato, R.O. Hynes, and D.D. Wagner. 2000. Persistence of platelet thrombus formation in arterioles of mice lacking both von Willebrand factor and fibrinogen. J Clin Invest 106:385.
41. Ault, K.A. and C. Knowles. 1995. In vivo biotinylation demonstrates that reticulated platelets are the youngest platelets in circulation. Exp Hematol 23:996.
42. Clemetson, K.J., J.L. McGregor, E. James, M. Dechavanne, and E.F. Luscher. 1982. Characterization of the platelet membrane glycoprotein abnormalities in Bernard-Soulier syndrome and comparison with normal by surface-labeling techniques and high-resolution two-dimensional gel electrophoresis. J Clin Invest 70:304.
43. Daeron, M. 1997. Fc receptor biology. Annu Rev Immunol 15:203.
44. Ravetch, J.V. 1997. Fc receptors. Curr Opin Immunol 9:121.
45. Sugiyama, T., M. Okuma, F. Ushikubi, S. Sensaki, K. Kanaji, and H. Uchino. 1987. A novel platelet aggregating factor found in a patient with defective collagen-induced platelet aggregation and autoimmune thrombocytopenia. Blood 69:1712.
46. Heemskerk, J.W., P. Siljander, W.M. Vuist, G. Breikers, C.P. Reutelingsperger, M.J. Barnes, C.G. Knight, R. Lassila, and R.W. Farndale. 1999. Function of glycoprotein VI and integrin alpha2beta1 in the procoagulant response of single, collagen-adherent platelets. Thromb Haemost 81:782.
47. Reverter, J.C., S. Beguin, H. Kessels, R. Kumar, H.C. Hemker, and B.S. Coller. 1996. Inhibition of platelet-mediated, tissue factor-induced thrombin generation by the mouse/human chimeric 7E3 antibody. Potential implications for the effect of c7E3 Fab treatment on acute thrombosis and "clinical restenosis". J Clin Invest 98:863.
48. Topol, E.J., T.V. Byzova, and E.F. Plow. 1999. Platelet GPIIb-IIIa blockers. Lancet 353:227.
49. Scarborough, R.M., N.S. Kleiman, and D.R. Phillips. 1999. Platelet glycoprotein IIb/IIIa antagonists. What are the relevant issues concerning their pharmacology and clinical use? Circulation 100:437.
50. Rodgers, R.P. and J. Levin. 1990. A critical reappraisal of the bleeding time. Semin Thromb Hemost 16:1.
51. Barnes, M.J., C.G. Knight, and R.W. Farndale. 1998. The collagen-platelet interaction. Curr Opin Hematol 5:314.
52. Moroi, M., I. Onitsuka, T. Imaizumi, and S.M. Jung. 2000. Involvement of activated integrin alpha2beta1 in the firm adhesion of platelets onto a surface of immobilized collagen under flow conditions. Thromb Haemost 83:769.
53. Watson, S., O. Berlanga, D. Best, and J. Frampton. 2000. Update on collagen receptor interactions in platelets: is the two-state model still valid? [In Process Citation]. Platelets 11:252.
54. Berlanga, O., R. Bobe, M. Becker, G. Murphy, M. Leduc, C. Bon, F.A. Barry, J.M. Gibbins, P. Garcia, J. Frampton, and S.P. Watson. 2000. Expression of the collagen receptor glycoprotein VI during megakaryocyte differentiation [In Process Citation]. Blood 96:2740.
55. Niedergang, F., A. Alcover, C.G. Knight, R.W. Farndale, M.J. Barnes, I.M. Francischetti, C. Bon, and M. Leduc. 2000. Convulxin binding to platelet receptor GPVI: competition with collagen related peptides. Biochem Biophys Res Commun 273:246.

**Table 1: Expression of glycoproteins and surface-bound fibrinogen on platelets from JAQ1-treated mice. Diluted whole blood from the indicated mice was incubated with FITC-labeled antibodies at saturating concentrations for 15 min at RT and platelets were analyzed directly. Results are expressed as mean log fluorescence ± S.D. for 6 mice per group.**

| | control | JAQ1 3d | JAQ1 7d | JAQ1 14d |
|---|---|---|---|---|
| *GPIIb*/*IIIa* | 321.3 ± 9.7 | 318.1 ± 9.4 | 328.7 ± 9.1 | 325.3 ± 9.8 |
| *GPIb-IX* | 278.9 ± 16.8 | 275.4 ± 18.0 | 269.5 ± 15.9 | 273.1 ± 11.4 |
| *GPV* | 165.4 ± 10.9 | 163.3 ± 14.1 | 169.1 ± 15.3 | 158.1 ± 10.5 |
| *CD9* | 543.8 ± 15.8 | 554.3 ± 14.6 | 549.5 ± 19.6 | 557.0 ± 13.0 |
| *GPIa (α2)* | 38.2 ± 6.7 | 40.3 ± 6.5 | 35.2 ± 7.8 | 36.7 ± 6.2 |
| *fibrinogen* | 14.1 ± 1.7 | 15.0 ± 1.4 | 14.3 ± 1.5 | 14.4 ± 1.5 |
| *P-selectin* | 6.2 ± 0.8 | 6.5 ± 0.8 | 6.7 ± 0.8 | 6.0 ± 1.1 |

### Legends to figures

### Figure 1: JAQ1 induces transient thrombocytopenia

Mice received purified IgG (a) or Fab fragments (b) of the indicated mAb i.p. in 200 µl sterile PBS. Platelet counts were determined at the indicated times using an improved Neubauer hemocytometer. Results are expressed as the mean platelet count ± SD for groups of each 6 mice.

### Figure 2: Platelets from JAQ1-treated mice do not respond to CRP and collagen

(a) Two color flow cytometric analysis of platelets from JAQ1-treated or control mice 3 days after antibody injection. Diluted whole blood was stimulated with 10 µM ADP or 10 µg/ml CRP for 2 min and subsequently incubated with anti-fibrinogen^{FITC} and anti-P-selectin^{PE} antibodies for 10 min at RT and analyzed directly. Platelets were gated by FSC/SSC characteristics and FI3 intensity (anti-mouse GPIbα^{PE/Cy5}). The data shown are representative of 6 mice per group. Similar results were obtained on days 7 and 14 after antibody injection.
(b) Heparinized prp from the indicated mice was stimulated with collagen (50 µg/ml), ADP (10 µM) or PMA (50 ng/ml). Light transmission was recorded on a Fibrintimer 4 channel aggregometer. (c) Heparinized prp from control mice was incubated with stirring in the presence of irrelevant rat IgG2a (20 µg/ml - circles) or JAQ1 (20 µg/ml - triangles) for 5 min before the addition of the indicated concentrations of collagen. In parallel, prp from JAQ1-treated mice was tested (squares). Results are expressed as the max. platelet aggregation ± S.D. for groups of each 6 mice.

### Figure 3: GPVI is not detectable in platelets from JAQ1-treated mice for at least two weeks

(a) Whole platelet proteins were separated by SDS-PAGE under non-reducing conditions and immunoblotted with FITC-labeled JAQ1 (anti-GPVI) or EDL1 (anti-GPIIIa). Bound mAb was detected by HRP-labeled rabbit anti-FITC and ECL. (b) Washed platelets from control, FcRγ chain-deficient (FcRγ -/-) and JAQ1-treated (day 7) mice were stimulated with 10 µg/ml convulxin (Cvx). Control platelets were pre-incubated with irrelevant rat IgG2a or JAQ1 (20 µg/ml) for 5 min before the addition of Cvx. (c) Washed platelets from the indicated mice were incubated with FITC-labeled convulxin (5 µg/ml) for 15 min at room temperature and then analyzed on a FACScan (Becton Dickinson). The data shown are representative of 6 mice per group.

### Figure 4: Reduced adhesion to collagen and abolished procoagulant response of GPVI-depleted platelets

(a) Platelets from JAQ1-treated mice (d 7) bind normal amounts of plasma vWF in the presence of botrocetin (2 µg/ml - solid line). Bound vWF was detected by FITC-labeled anti-vWF antibodies (10 µg/ml). No binding was detected in the absence of botrocetin (shaded area). Normal activation of β1-integrins on platelets from JAQ1-treated mice in response to thrombin (0.1 U/ml). Resting (shaded area) or thrombin activated (solid line) platelets were incubated with FITC-labeled 9EG7 (5 µg/ml) for 15 min at RT and analyzed directly. (b) Washed platelets from control or JAQ1-treated mice (d 7) were incubated in collagen-coated microtiter plates in the presence or absence of MgCl₂ (1 mM) /CaCl₂ (1 mM) for the indicated times and adherent platelets were quantitated fluorimetrically. The data shown are from a single experiment, representative of five identical experiments and expressed as the mean of triplicate readings ± SD. (c) Flow cytometric analysis of Annexin V-FITC binding to platelets from control and JAQ1-treated (d 7) mice activated with a combination of collagen (50 µg/ml) and thrombin (0.01 U/ml).

### Figure 5: Bleeding time of JAQ1-treated mice

Bleeding times were determined in mice 7 days after injection of 100 µg non-immune IgG2a or JAQ1 (n=15 per group). As a control, mice received 100 µg F(ab)₂ fragments of JON/A (anti-GPIIb/IIIa) 24 h before the experiment (n=6). Where necessary, bleeding was manually stopped at the 10 min-time point to prevent death. Each point represents one individual.

### Figure 6: JAQ1 induces long-term protection from intravascular thrombosis

Thromboembolism in response to a bolus injection of a mixture of collagen (0.8 mg/kg body weight) and epinephrine (60 µg/kg body weight). (a) Mortality in control mice and mice treated with 100 µg JAQ1 at the indicated times before challenge. (b) Platelet counts in control and JAQ1-treated mice 3 min after infusion of collagen/epinephrine (n=8 per group). (c) Upper panel: representative histology of the lungs (original x 100); obstructed vessels are indicated by arrows. Lower panel: immunohistochemical detection of platelets in the thrombi (original x 400). Acetone fixed frozen sections were reacted with a platelet-specific antibody (anti-GPIb-IX) and counterstained with hematoxylin. The red horseradish peroxidase reaction product shows high density of platelets in the thrombus.

## Claims

1. Medicament for the protection against thrombotic diseases, **characterized in that** it comprises an antibody, which induces an irreversible inactivation or degradation of the collagen receptor on thrombocytes and binds to the same epitop of the collagen receptor GPVI on thrombocytes as the monoclonal antibody JAQ1, obtainable from the hybridoma cell line with the deposition number DSM ACC 2487.

2. Medicament as claimed in claim 1, **characterized in that** the monoclonal antibody is JAQ1.

3. Medicament as claimed in claims 1 or 2, **characterized in that** it contains the humanised monoclonal antibody JAQ1.

4. A diagnostic agent for the determination of the expression rate of the collagen receptor GPVI, **characterized in that** it contains a labelled monoclonal or polyclonal antibody directed against the same GPVI epitope as defined by JAQ1, obtainable from the hybridoma cell line with the deposition number DSM ACC 2487.

5. A method for the determination of the expression rate of the collagen receptor GPVI in blood **characterized in that**
a) a sample of the blood of the patient is incubated with a solid carrier on which the antibody JAQ1, obtainable from the hybridoma cell line with the deposition number DSM ACC 2487, is fixed, washing the carrier, incubating it with a second labelled antibody JAQ1, washing the carrier again and measuring the signal of the second labelled antibody; or
b) a sample of the blood of the patient is fixed on a solid carrier and thereafter treated with the labelled antibody JAQ1, obtainable from the hybridoma cell line with the deposition number DSM ACC 2487, alone or in mixture with the unlabelled antibody JAQ1 and subsequently the labelled antibody is detected; or
c) the monoclonal antibody JAQ1, obtainable from the hybridoma cell line with the deposition number DSM ACC 2487, is fixed on a solid carrier and is thereafter contacted with the blood sample, which is to be tested, together with the labelled antibody JAQ1, washing the carrier and measuring the signal of the labelled antibody.

6. A method as claimed in claim 5, **characterized in that** it is performed using a fluorescence-labelled monoclonal JAQ1 antibody in a flow-cytometer.

7. A hybridoma cell line for the production of the monoclonal antibody JAQ1 which cell line carries the deposition number DSM ACC 2487.

8. Monoclonal antibody, **characterized in that** it binds to the same epitop of the collagen receptor on thrombocytes as the monoclonal antibody JAQ1, obtainable from the hybridoma cell line with the deposition number DSM ACC 2487.

9. Use of an antibody, which induces an irreversible inactivation or degradation of the collagen receptor on thrombocytes and binds to the same epitop of the collagen receptor GPVI on thrombocytes as the monoclonal antibody JAQ1, obtainable from the hybridoma cell line with the deposition number DSM ACC 2487, for the preparation of a medicament against thrombotic diseases.

10. Use as claimed in claim 9, wherein the antibody is a monoclonal antibody.

11. Use as claimed in claims 9 and 10, wherein the antibody is the monoclonal antibody JAQ1.

## Patentansprüche

1. Medikament zum Schutz gegen thrombotische Krankheiten, **dadurch gekennzeichnet, dass** es einen Antikörper enthält, der eine irreversible Inaktivierung oder einen irreversiblen Abbau des Kollagenrezeptors auf Blutplättchen induziert und sich an das gleiche Epitop des Kollagenrezeptors GPVI auf Blutplättchen bindet, wie der monoklonale Antikörper JAQ1, der aus der Hybridom-Zellinie mit der Hinterlegungsnummer DSM ACC 2487 erhältlich ist.

2. Medikament nach Anspruch 1, **dadurch gekennzeichnet, dass** es sich bei dem monoklonalen Antikörper um JAQ1 handelt.

3. Medikament nach Anspruch 1 oder 2, **dadurch gekennzeichnet, dass** es den humanisierten monoklonalen Antikörper JAQ1 enthält.

4. Diagnostikum zur Bestimmung der Expressionsrate des Kollagenrezeptors GPVI, **dadurch gekennzeichnet, dass** es einen markierten monoklonalen oder polyklonalen Antikörper enthält, der auf das gleiche GPVI-Epitop zielt wie durch JAQ1 definiert, erhältlich aus der Hybridom-Zellinie mit der Hinterlegungsnummer DSM ACC 2487.

5. Verfahren zur Bestimmung der Expressionsrate des Kollagenrezeptors GPVI in Blut, **dadurch gekennzeichnet, dass** man
a) eine Probe des Bluts des Patienten mit einem festen Träger inkubiert, auf dem der Antikörper JAQ1, erhältlich aus der Hybridom-Zellinie mit der Hinterlegungsnummer DSM ACC 2487, fixiert ist, den Träger wäscht, ihn mit einem zweiten markierten Antikörper JAQ1 inkubiert, den Träger nochmals wäscht und das Signal des zweitmarkierten Antikörpers mißt; oder
b) eine Probe des Bluts des Patienten auf einem festen Träger fixiert und anschließend mit dem markierten Antikörper JAQ1, erhältlich aus der Hybridom-Zellinie mit der Hinterlegungsnummer DSM ACC 2487, alleine oder in einer Mischung mit dem nichtmarkierten Antikörper JAQ1 behandelt und anschließend den markierten Antikörper nachweist; oder
c) den monoklonalen Antikörper JAQ1, erhältlich aus der Hybridom-Zellinie mit der Hinterlegungsnummer DSM ACC 2487, auf einem festen Träger fixiert und anschließend mit der zu testenden Blutprobe zusammen mit dem markierten Antikörper JAQ1 in Kontakt bringt, den Träger wäscht und das Signal des markierten Antikörpers mißt.

6. Verfahren nach Anspruch 5, **dadurch gekennzeichnet, dass** es mit einem fluoreszenzmarkierten monoklonalen JAQ1-Antikörper in einem Durchflußzytometer durchgeführt wird.

7. Hybridom-Zellinie zur Produktion des monoklonalen Antikörpers JAQ1, dessen Zellinie die Hinterlegungsnummer DSM ACC 2487 hat.

8. Monoklonaler Antikörper, **dadurch gekennzeichnet, dass** er an das gleiche Epitop des Kollagenrezeptors auf Blutplättchen bindet wie der monoklonale Antikörper JAQ1, der aus der Hybridom-Zellinie mit der Hinterlegungsnummer DSM ACC 2487 erhältlich ist.

9. Verwendung eines Antikörpers, der eine irreversible Inaktivierung oder einen irreversiblen Abbau des Kollagenrezeptors auf Blutplättchen induziert und an das gleiche Epitop des Kollagenrezeptors GPVI auf Blutplättchen bindet wie der monoklonale Antikörper JAQ1, erhältlich aus der Hybridom-Zellinie mit der Hinterlegungsnummer DSM ACC 2487, zur Herstellung eines Medikaments gegen thrombotische Krankheiten.

10. Verwendung nach Anspruch 9, wobei es sich bei dem Antikörper um einen monoklonalen Antikörper handelt.

11. Verwendung nach Anspruch 9 oder 10, wobei es sich bei dem Antikörper um den monoklonalen Antikörper JAQ1 handelt.

## Revendications

1. Médicament pour la protection contre les maladies thrombotiques, **caractérisé en ce qu'**il comprend un anticorps qui induit une inactivation ou une dégradation irréversibles du récepteur du collagène sur les thrombocytes et qui se lie au même épitope du récepteur du collagène GPVI sur les thrombocytes que l'anticorps monoclonal JAQ1, que l'on peut obtenir à partir de la lignée de cellules d'hybridome ayant le numéro de dépôt DSM ACC 2487.

2. Médicament, selon la revendication 1, **caractérisé en ce que** l'anticorps monoclonal est le JAQ1.

3. Médicament, selon la revendication 1 ou la 2, **caractérisé en ce qu'**il contient l'anticorps monoclonal humanisé JAQ1.

4. Agent diagnostique pour la détermination du niveau d'expression du récepteur du collagène GPVI, **caractérisé en ce qu'**il contient un anticorps monoclonal ou polyclonal marqué dirigé contre le même épitope de GPVI que celui défini par JAQ1, que l'on peut obtenir à partir de la lignée de cellules d'hybridome ayant le numéro de dépôt DSM ACC 2487.

5. Procédé pour la détermination du niveau d'expression du récepteur du collagène GPVI dans le sang, **caractérisé en ce que** :
a) un échantillon du sang du patient est incubé avec un transporteur solide sur lequel est fixé l'anticorps JAQ1, que l'on peut obtenir à partir de la lignée de cellules d'hybridome ayant le numéro de dépôt DSM ACC 2487, on lave le transporteur, on l'incube avec un deuxième anticorps JAQ1 marqué ; on lave à nouveau le transporteur et on mesure le signal du deuxième anticorps marqué ; ou
b) un échantillon du sang du patient est fixé sur un transporteur solide et est traité par la suite avec l'anticorps JAQ1 marqué, que l'on peut obtenir à partir de la lignée de cellules d'hybridome ayant le numéro de dépôt DSM ACC 2487, seul ou en mélange avec l'anticorps JAQ1 non marqué, et ensuite on détecte l'anticorps marqué ; ou
c) l'anticorps monoclonal JAQ1, que l'on peut obtenir à partir de la lignée de cellules d'hybridome ayant le numéro de dépôt DSM ACC 2487, est fixé sur un transporteur solide et est mis en contact par la suite avec l'échantillon de sang devant être testé conjointement avec l'anticorps JAQ1 marqué, on lave le transporteur et on mesure le signal de l'anticorps marqué.

6. Procédé selon la revendication 5, **caractérisé en ce qu'**il est exécuté en utilisant un anticorps monoclonal JAQ1 marqué par fluorescence dans un cytomètre en flux.

7. Lignée de cellules d'hybridome destinée à la production de l'anticorps monoclonal JAQ1, laquelle lignée de cellules porte le numéro de dépôt DSM ACC 2487.

8. Anticorps monoclonal **caractérisé en ce qu'**il se lie au même épitope du récepteur du collagène sur les thrombocytes que l'anticorps monoclonal JAQ1, que l'on peut obtenir à partir de la lignée de cellules d'hybridome ayant le numéro de dépôt DSM ACC 2487.

9. Utilisation d'un anticorps qui induit une inactivation ou une dégradation irréversibles du récepteur du collagène sur les thrombocytes et qui se lie au même épitope du récepteur du collagène GPVI sur les thrombocytes que l'anticorps monoclonal JAQ1, que l'on peut obtenir à partir de la lignée de cellules d'hybridome ayant le numéro de dépôt DSM ACC 2487, destiné à la préparation d'un médicament contre les maladies thrombotiques.

10. Utilisation selon la revendication 9, dans laquelle l'anticorps est un anticorps monoclonal.

11. Utilisation selon la revendication 9 ou la 10, dans laquelle l'anticorps est l'anticorps monoclonal JAQ1.
